Europäisches Patentamt

European Patent Office

Office européen des brevets

(11) Publication number: **0 271 029 B1**

# EUROPEAN PATENT SPECIFICATION

(45) Date of publication of patent specification: **28.07.93**  (51) Int. Cl.⁵: **A61M  15/00**

(21) Application number: **87117992.5**

(22) Date of filing: **04.12.87**

㊴ Inhaler.

(30) Priority: **05.12.86 JP 291163/86**

(43) Date of publication of application:
**15.06.88 Bulletin  88/24**

(45) Publication of the grant of the patent:
**28.07.93 Bulletin  93/30**

(84) Designated Contracting States:
**AT BE CH DE ES FR GB GR IT LI LU NL SE**

(56) References cited:
| | |
|---|---|
| **EP-A- 0 015 247** | **EP-A- 0 074 937** |
| **GB-A- 1 118 341** | **GB-A- 2 064 336** |
| **US-A- 3 809 084** | **US-A- 4 192 309** |

(73) Proprietor: **MECT CORPORATION**
**1-1, Nishishinjuku 2-Chome**
**Shinjuku-ku Tokyo 163(JP)**

(72) Inventor: **Abiko, Kenji**
**273-26, Aza Sagigahukuro Kitamachi**
**Kaminoyama-shi Yamagata(JP)**
Inventor: **Minagawa, Shikoh**
**11-7, Motoki 1-chome**
**Yamagata-shi Yamagata(JP)**
Inventor: **Shinohara, Hiroshi**
**538-2, Ochiaicho**
**Yamagata-shi Yamagata(JP)**
Inventor: **Koseki, Kayoko**
**531-1, Koriyama**
**Nanyo-shi Yamagata(JP)**

(74) Representative: **Dr. Elisabeth Jung Dr. Jürgen**
**Schirdewahn Dipl.-Ing. Claus Gernhardt**
**P.O. Box 40 14 68 Clemensstrasse 30**
**W-8000 München 40 (DE)**

Rank Xerox (UK) Business Services
(3. 10 / 3.6 / 3.3. 1)

## EP 0 271 029 B1

**Description**

This invention relates to inhalers, particularly to an inhaler effective in use for inhaling pharmaceutical powdered preparations and making the same reach a trachea, bronchi and pulmonary cells.

Related Art Statement

In general, in order to distribute pharmaceutical preparations into an oral cavity of the human body to reach the trachea, bronchi and pulmonary cells of a human body, there has heretofore been used an atomizing type spraying device. However, with the device of this type, patients tend to close the deep portion of the oral cavity instinctively or in a conditioned response at the moment of spraying, so that it has been difficult to smoothly achieve an expected purpose.

Therefore, to obviate the above-described disadvantage, there have been proposed inhalers for smoothly inhaling the pharmaceutical powdered preparations into the trachea, bronchi and pulmonary cells in accompany with the inspiration of intake air by a patient.

As one of these inhalers, in Japanese Patent Laid-Open No. 53-136392, there has been disclosed an inhaler comprising: a straight-lined hollow barrel body formed therein with a chamber and provided at a side surface thereof with air flow-in openings communicated with the chamber; and a lid sleeve connected to one end of this barrel body, having a capsule holding sleeve and rotatable relative to the barrel body; wherein, when the barrel body and the lid sleeve are rotated relative to each other, a capsule insertion portion held by the capsule holding sleeve is engaged with an abutting portion in the barrel body, whereby the capsule is divided into two including a capsule barrel and a capsule head, so that the powdered preparations contained in the capsule is dropped into the chamber and inhaled into an oral cavity of the patient through a nozzle.

Furthermore, in GB-A-2 064 336 and in the magazine "The Practitioner" March, 1982 issue, 226th volume, pages 565 - 567, there is disclosed an inhaler showing the precharacterizing features of claim 1, among others, comprising: a straight-lined hollow inner barrel; and an outer barrel connected to one end of the inner barrel and rotatable relative to the inner barrel, said outer barrel being provided at one end thereof with a capsule insertion hole and air flow-in openings; wherein the inner barrel is rotated relative to the outer barrel, whereby a capsule insertion portion is engaged with an abutting portion in the inner barrel, so that a capsule is divided into two including a main body and a head and powdered preparations which have fallen into the inner barrel are inhaled from the other end of the inner barrel.

However, it has been clarified by the studies made by the inventors of the present invention that the above-described two inhalers of the prior art present serious problems when used as the inhaler.

First, the former, i.e. the inhaler of the prior art disclosed in the Japanese Patent Laid-Open No. 53-136392 presents the following problems.

(1) The inhaler as a whole is of a straight-lined form, and, when the capsule is divided into two including the main body and the head thereof by the rotation of the hollow barrel body and the lid sleeve relative to each other, the powdered preparations which have fallen into the hollow barrel body may leak out of the hollow barrel body to the outside. Because of this, shortage or waste of the contained material may be caused. This problem is particularly notable because the capsule insertion hole is positioned at a location very close to the nozzle for inhaling.

(2) The air flow-in openings for the hollow barrel body are formed in the side surface, and the end face is blocked, whereby ideal turbulent flows are difficult to be formed in the chamber and the operating manoeuvres of the capsule barrel which has fallen into the chamber are not briskly performed. Accordingly, the powdered preparations in the capsule barrel are difficult to be discharged into the hollow barrel body, and further, the powdered preparations which have fallen into the chamber cannot be efficiently inhaled out of the chamber, so that the powdered preparations remain in the capsule, and moreover, in the chamber. Therefore, an expected remedial result cannot be achieved due to the shortage of the planned dosage.

(3) Since the capsule holding sleeve is provided at a position very close to the inhaling nozzle, the capsule disturbs the patient when he performs inhalation with the inhaling nozzle being put in his mouth.

(4) Since the general form of the inhaler is of a straight-lined cylindrical form, there is a possibility of that the inhaler turns to fall down from a table when the inhaler is placed on a flat plane such as a table.

Possibly due to the above-described serious disadvantages, the inhaler of this type has not heretofore been put on the market as a product.

Next, the latter inhaler of the prior art presents the following serious drawbacks.

(a) The inhaler as a whole is of a straight-lined form and the powdered preparations, which have fallen from the capsule into the inner barrel by the rotation of the inner barrel and the outer barrel relative to each other, leak out of the inner barrel to the outside as in the case of the former invention of the prior art in the above Item (1). As the result, an insufficient or wasteful dosage of the powdered preparations is caused.

(b) The air flow-in openings for the inhaler are formed formed in the end face of the outer barrel, however, the inhaler as a whole is of a straight-lined form, whereby ideal turbulent flows are difficult to be formed in the inhaler during inhaling and the operating manoeuvres of the capsule barrel which has fallen into the inhaler are not briskly performed. Accordingly, the powdered preparations in the capsule barrel are not smoothly discharged, and moreover, the powdered preparations are not smoothly inhaled through the inhaling nozzle and remain in the inhaler. Besides, in the latter invention of the prior art, there is not disclosed that at what positions air flow-in openings having what shape and what size should be provided.

(c) In connection with Item (b), ideal turbulent flows are difficult to be formed in the inhaler, whereby the operating manoeuvres of the capsule barrel which has fallen into the inhaler are poor, and the powdered preparations in the capsule barrel are difficult to go out of the capsule barrel and remain therein. Because of this, an insufficient remedial result is caused due to a shortage of the planned dosage.

(d) Furthermore, it has been clarified by the experiments made by the inventors of the present invention that the capsule head still held in the capsule insertion hole of the outer barrel after the capsule barrel is separated therefrom is open at the opening end thereof to the inhaler, whereby, to everyone's surprise, the powdered preparations in the inhaler are recirculated and received again in the capsule head by air streams generated in the inhaler and remain in the capsule head as they are.

(e) Since the general form of the inhaler is of a straight-lined cylindrical form, there is a possibility of that the inhaler turns to fall down from a table when the inhaler is placed on a flat plane such as a table.

## SUMMARY OF THE INVENTION

The present invention has been developed by the earnest studies in view of the above-described problems of the prior art and has as one of its objects the provision of an inhaler for allowing efficient inhalation with no material of the capsule remaining in the inhaler, capsule head and capsule barrel.

Another object of the present invention is to provide an inhaler capable of preventing the material contained in the capsule from leaking out of an inhaling section when the capsule set in the capsule insertion hole is divided into two including the barrel and the head.

The above objects are reached by an inhaler having the features of claim 1.

In an inhaler according to the present invention the hollow body and the capsule holding portion are rotated relative to each other to divide the capsule into two sections including the capsule barrel and the capsule head, whereby the material contained in the capsule is made to fall into the inhaler, said hollow body includes a hollow barrel section having a predetermined length and a hollow bent section connected to one end of the hollow barrel section and having an axis inclined at a predetermined angle to an axis of the hollow barrel section.

With the above-described arrangement, the hollow body is of non-straight-lined form, whereby the general form of the inhaler becomes bent, so that the turbulent flows are readily formed in the inhaler during inhaling. Accordingly, the material contained in the capsule, which has fallen into the inhaler, is smoothly inhaled to the outside of the inhaler, and does not remain in the inhaler and the capsule, so that an expected inhaling value can be obtained.

Furthermore, the material contained in the capsule can be effectively prevented from leaking out of the inhaler when the capsule is divided, owing to the bent construction of the inhaler.

From GB-A-1 118 341 already an inhaler is known in which a bent section is connected with a straight section by a fixed angle of 90°. An air inlet can consist of several small holes the total cross-sectional area of which is substantially equal to that of the mouthpiece opening. Thus, a turbulent stream of air is caused intended to be capable of causing break-up and/or dispersion of powder which may be present in the bowl having means for holding a container of powdered medicament.

To connect a straight section with a bent section by an angle is known per se from US-A-4 192 309 (particularly Fig. 2) and EP-A-0 074 937 (particularly Fig. 1).

## BRIEF DESCRIPTION OF THE DRAWINGS

Other objects and merits of the present invention will be apparent from the following description made with reference to the preferred embodiments shown in the drawings, in which:

Fig. 1 is a sectional view showing the inhaler in an embodiment 1 of the present invention, taken along the line I - I in Fig. 2;

Fig. 2 is a plan view thereof;

Fig. 3 is a front view thereof;

Fig. 4 is a left side view thereof;

Figs. 5(a), 5(b), 5(c) are views showing embodiments of forms and arrangements of air flow-in openings usable in the present invention, respectively;

Figs. 6(a), 6(b), 6(c) and 6(d) are views showing various comparative examples of forms and arrangements of air flow-in openings, respectively;

Figs. 7(a) and 7(b) are views showing the positional relationship between the inhaler and the air flow-in openings during inhaling, respectively;

Fig. 8 is a sectional view showing another embodiment of the inhaler according to the present invention; and

Fig. 9 is a front view thereof.

## DETAILED DESCRIPTION OF THE PREFERRED EMBODIMENTS

[Embodiment 1]

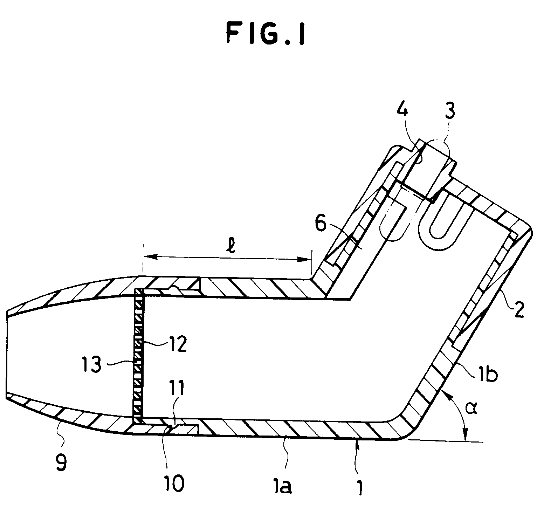

Referring now to the drawings, as an example, the inhaler according to this embodiment as a whole can be made of an opaque synthetic resin material. This inhaler has a hollow body 1 including a hollow barrel section 1a having a predetermined length $\ell$ as indicated at the upper position in Fig. 1 and a straight-lined hollow bent section 1b formed integrally with this hollow barrel section 1a and having an axis inclined at a predetermined angle $\alpha$ to an axis of the hollow barrel section 1a.

A generally cup-shaped capsule holding portion 2 formed of a molded part made of a synthetic resin material for example is mounted on one end of the bent section 1b of the hollow body 1 in a manner to be rotatable relative to the hollow body 1. As indicated by two-dot chain lines in Fig. 1, formed at the end face of this capsule holding portion 2 are a generally square-shaped capsule insertion hole 4 for receiving and holding therein a capsule 3 containing therein a material such for example as powdered preparations and two generally arcuate slit-shaped air flow-in openings 5 for allowing intake air to flow into the inhaler.

A capsule head as being a large diametral side of the capsule 3 is inserted into the capsule insertion hole 4 and held thereby, and a capsule barrel as being a smaller diametral side of the capsule 3 projects into the bent section 1b of the hollow body 1. An abutting ridge (abutting means) 6 extending at least to a position capable of abutting against the capsule 3's portion inserted and projected into the bent section 1b, i.e. the capsule barrel, when the capsule 3 is inserted into the capsule insertion hole 4 and held thereby, is projectingly provided and formed integrally at a portion of the inner surface of the bent section 1b of the hollow body 1. This is because, when the capsule holding portion 2 is rotated relative to the hollow body 1, the abutting ridge 6 abuts against the capsule 3's portion inserted and projected into the inhaler, i.e. the capsule barrel, whereby the capsule barrel is separated from the capsule head, so that the powdered preparations in the capsule 3 can fall into the inhaler. Furthermore, to restrict a value of rotation between the hollow body 1 and the capsule holding portion 2 to a predetermined angle, in this embodiment, a raised portion 7 formed at the tail end of the bent section 1b is coupled into a groove 8 formed at one end of the capsule holding portion 2 for a predetermined length in the circumferential direction of the capsule holding portion 2. With this arrangement, the hollow body 1 and the capsule holding portion 2 are rotatable relative to each other within a value for which the raised portion 7 can turn in the groove 8 in the rotating direction. However, such an arrangement may be adopted that the hollow body 1 and the capsule holding portion 2 are freely rotatable relative to each other over an angle of 360 degrees without providing the raised portion 7 and the groove 8 as described above.

On the other hand, detachably, couplingly connected to an end portion of the hollow barrel section 1a of the hollow body 1 is a hollow inhaling section 9 to be held in the mouth of the patient when the patient sucks out together with intake air and inhales the powdered preparations which have fallen from the capsule into the inhaler. The inhaling section 9 is detachably connected to the hollow body 1 by coupling at a stroke an annular groove 11 on the inner periphery of a thin wall portion of the inhaling section 9 onto an annular ridge 10 on the outer periphery of a thin wall portion of the hollow body 1. The outer shape of the inhaling

4

section 9 is tapered toward the forward end so that the patient can easily put the inhaling section in his mouth.

Furthermore, a porous plate (capsule discharging preventive means) 12 formed of a molded part made of a synthetic resin material for example is connected to an end face of the hollow body 1 on the side of the inhaling section 9 through adhesive bonding for example. This porous plate 12 has a multitude of pores 13 for ventilating. The pores 13 may have such a size as to allow the powdered preparations as being the material contained in the capsule 3 to pass therethrough freely but not to allow the capsule barrel which has fallen into the hollow body 1 to be discharged to the outside of the inhaler. The porous plate 12 need not necessarily be limited to the molded part made of synthetic resin material, and may be a screen-like woven material for example.

Description will hereunder be given of action of this embodiment.

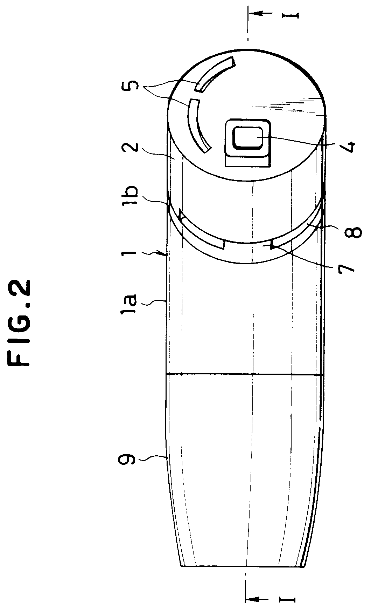

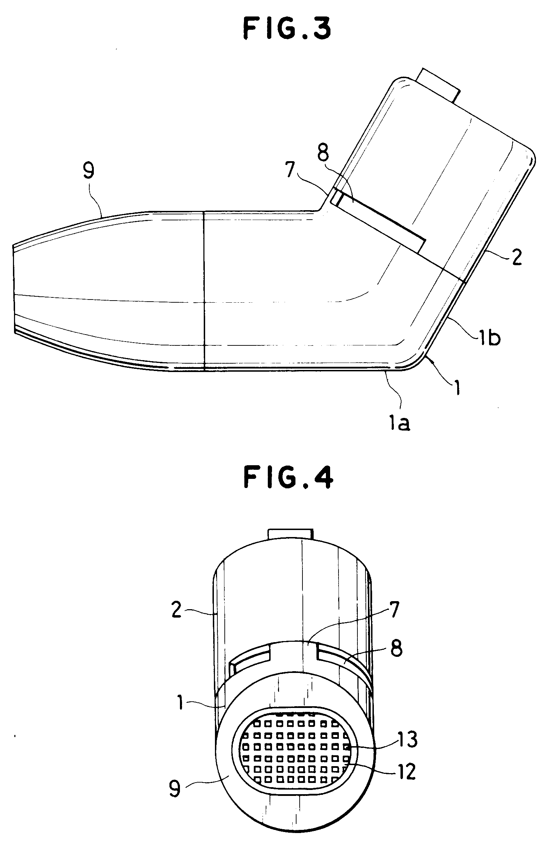

First, in the state where the hollow body 1 of the inhaler, the capsule holding portion 2, the inhaling section 9 and the porous plate 12 are assembled to one another as shown in Figs. 1 to 4, the capsule 3 containing therein the powdered preparations is inserted into the capsule insertion hole 4 of the capsule holding portion 2. At this time, the capsule head is held by the capsule insertion hole 4, and the capsule barrel is inserted and projected into the bent section 1b in the axial direction thereof in such a manner that at least the forward end of the capsule barrel is inserted and projected into the bent section 1b of the hollow body 1 in the axial direction thereof by a dimension capable of satisfactorily abutting against the abutting ridge 6 of the bent section 1b.

When, in this state, the patient holds the hollow barrel section 1a of the hollow body 1 and the capsule holding portion 2 in his right and left hands, respectively, and rotates the hollow body 1 and the capsule barrel relative to each other, the capsule barrel being the capsule 3's portion inserted and projected is abutted against the abutting ridge 6 of the hollow body 1. With this operation, the capsule 3 receives a force for separating the capsule barrel from the capsule head, whereby the capsule barrel is separated from the capsule head held by the capsule insertion hole 4 of the capsule holding portion 2 and only the capsule barrel falls into the hollow body 1. At this time, at least part of the powdered preparations in the capsule 3 is discharged from the capsule barrel into the hollow body 1 of the inhaler. However, the powdered preparations discharged into the hollow body 1 is prevented from leaking to the outside of the hollow body 1 because the hollow body 1 is of a bent form.

Subsequently, when the patient holds the inhaler, applies his mouth to the tapered portion of the inhaling section 9 to put it into his mouth and inhales, external air flows into the inhaler through the air flow-in openings 5 of the capsule holding portion 2 due to the inhaling force of the patient at that time, the powdered preparations in the inhaler is inhaled into the oral cavity of the patient together with the intake air through the pores 13 of the porous plate 12, and adhere to the trachea, bronchi and pulmonary cells of the patient. Then, the intake air, after being taken in through the air flow-in openings 5, is turned into a turbulent flow state in the hollow body 1 of the inhaler. This turbulent flow state can be very smoothly and readily formed because the inhaler is of the bent form.

As a consequence, due to the turbulent flows, the capsule barrel, which has fallen into the hollow body 1, striking the inner wall surface of the hollow body 1 from various directions and rebounding in the hollow body 1, so that the powdered preparations remaining in the capsule barrel are discharged into the hollow body 1 and inhaled into the oral cavity of the patient from the hollow body 1 through the porous plate 12 and the inhaling section 9. Additionally, the capsule barrel which has fallen into the hollow body 1 is not passed through the pores of the porous plate 12 and is prevented from being sucked out of the inhaler together with the intake air.

As described above, in this embodiment, the hollow body 1 of the inhaler has such a bent construction that the hollow barrel section 1a and the bent section 1b are inclined at the angle $\alpha$ to each other, so that the turbulent flows in the inhaler can be readily and reliably formed and the powdered preparations in the capsule 3 can be reliably inhaled into the oral cavity of the patient from the inhaler. Accordingly, the powdered preparations do not remain in the inhaler and the capsule and are effectively inhaled into the oral cavity of the patient, so that an insufficient dosage and wasteful preparations can be prevented from occurring.

Now, it has been found by the empirical studies made by the inventors of the present invention that the following arrangements make the inhaler according to the present invention particularly useful.

First, as for the angle $\alpha$ made by the axes of the hollow barrel section 1a and the bent section 1b of the hollow body 1, the operating manoeuvres of the capsule barrel in the inhaler are not brisk when the angle $\alpha$ is 90 degrees, the functional effect as being of the bent form can be achieved when the angle $\alpha$ is within the range from 30 to 80 degrees, and the operating manoeuvres of the capsule barrel become bricker when the angle $\alpha$ is within the range from 45 to 60 degrees. Particularly, when the angle $\alpha$ was set at 60 degrees, the turbulent flows were reliably formed in the hollow body 1, whereby the operating manoeuvres of the capsule barrel in the hollow body 1 were performed very briskly, so that it was possible to inhale the powdered preparations in the inhaler efficiently for a short period of time. Moreover, by setting the angle $\alpha$ at the values as described above, such problems were effectively prevented from occurring that the powdered preparations in the hollow body 1 were recirculated into the capsule head held by the capsule insertion hole 4 and remained therein. Additionally, when the angle $\alpha$ was set at a value smaller than 30 degrees, the disadvantages inherent to the conventional straight-lined inhaler were presented.

As for the length of the hollow barrel section 1a of the hollow body 1 of the inhaler; i.e. a length $\ell$ from one end of the hollow barrel section 1a to a boundary portion between the hollow barrel section 1a and the bent section 1b on the inner side in the bending direction of the bent section 1b, this length $\ell$ was preferably made to be 1.5 - 3.5 cm, and particularly, it was found that, by making the length $\ell$ to be 2.5 - 3.0 cm, the operating manoeuvres of the capsule barrel, the inhaling efficiency of the powdered preparations and the like were highly satisfactory. The inner diameter of the hollow body 1 in that case was about 2.1 cm for an example.

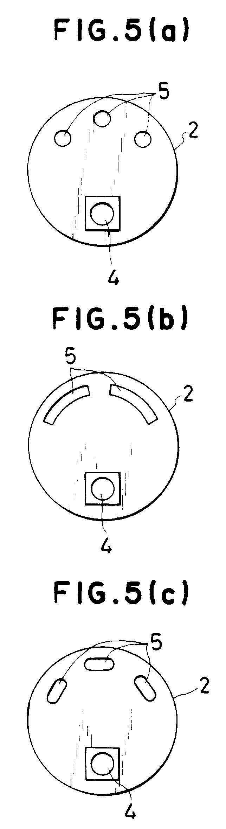

Further, when the inventors of the present invention studied the forms and arrangements of the air flow-in openings 5, the following results were obtained. Description will hereunder be given of these results with reference to Figs. 5 to 7.

More specifically, Figs. 5(a), 5(b) and 5(c) show various embodiments of the air flow-in openings applicable to the present invention, respectively.

First, the air flow-in openings 5 in the embodiment shown in Fig. 5(a) are constructed such that three circular holes are arranged arcuately with one another at regular intervals along and close to the outer periphery of an end face of the capsule holding portion 2 at positions substantially diametrically opposite to the capsule insertion hole 4. The diameter of each flow-in opening 5 is 1.5 - 4.0 mm, preferably about 3.5 mm. The inhaler of this type is indicated as an L type in TABLE 1.

6

TABLE 1   COMPARISONS OF POWDER SCATTERING PROPERTIES BETWEEN INHALERS

| INHALERS OF THIS INVENTION | | NO. OF SAMPLES n | RATE OF REMAINING IN INHALER | RATE OF REMAINING IN CAPSULE HEAD | RATE OF REMAINING IN CAPSULE BARREL | RATE OF SCATTERING [%] |
|---|---|---|---|---|---|---|
| L TYPE | LONG BARREL | 3 | 7.6 ±0.84 | 0.3 ±0.05 | 3.0 ±0.22 | 89.1 ±0.68 |
| | SHORT BARREL | 3 | 7.8 ±1.25 | 0.3 ±0.03 | 2.9 ±0.43 | 89.0 ±0.79 |
| A TYPE | LONG BARREL | 3 | 8.1 ±0.40 | 0.2 ±0.01 | 1.9 ±0.30 | 89.8 ±0.30 |
| | SHORT BARREL | 3 | 8.1 ±0.73 | 0.1 ±0.07 | 1.3 ±0.26 | 90.5 ±0.54 |
| S TYPE | LONG BARREL | 3 | 8.6 ±0.26 | 0.3 ±0.01 | 1.8 ±0.08 | 89.3 ±0.24 |
| | SHORT BARREL | 3 | 8.5 ±0.77 | 0.4 ±0.26 | 2.0 ±0.33 | 89.1 ±0.69 |
| PRIOR ART | | 3 | 22.8 ±0.68 | 4.7 ±0.40 | 3.6 ±0.63 | 68.9 ±0.44 |

The embodiment shown in Fig. 5(b) is constructed such that two arcuate slit-shaped air flow-in openings 5 similar to the embodiments shown in Figs. 1 - 4 are arranged arcuately with one another at positions diametrically opposite to the capsule insertion hole 4, and the positional relationship between the air flow-in openings 5 and the capsule insertion hole 4 is different from ones shown in Figs. 1 - 4. The inhaler of this type is indicated as an A type in TABLE 1.

7

The embodiment shown in Fig. 5(c) is constructed such that three slot- or tablet-shaped air flow-in openings 5 are arranged arcuately with one another at positions diametrically opposite to the capsule insertion hole similarly to the case of the circular holes in Fig. 5(a). The inhaler of this type is indicated as an S type in TABLE 1.

TABLE 1 shows examples wherein these three type inhalers having the constructions of the air flow-in openings according to the present invention, i.e. the L type, A type and S type inhalers, are compared with the latter of the aforesaid inhalers of the prior art. As apparent from TABLE 1, each of the inhalers according to the present invention each has a low rate at which the powdered preparations remain in the inhaler and the capsule as compared with the prior art, so that a highly satisfactory rate of scattering, i.e. a highly satisfactory rate of inhaling to the outside of the inhaler can be attained.

Subsequently, the inventors of the present invention conducted experiments on the influences rendered to the powder scattering by the forms and arrangements of the air flow-in openings 5.

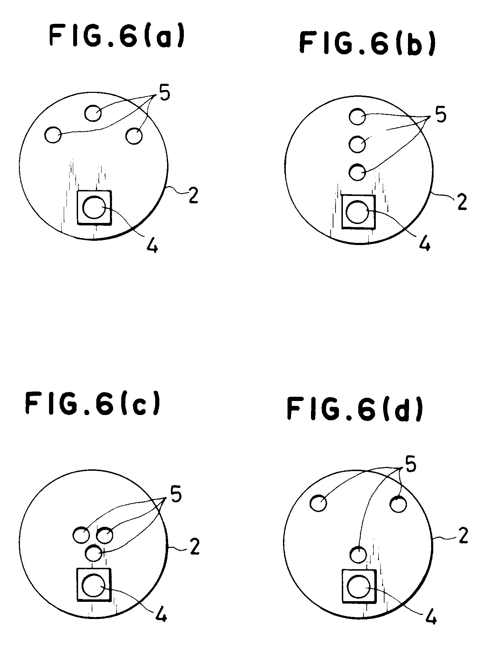

The forms of the air flow-in openings 5 studied under the experiments are shown in Figs. 6(a), 6(b), 6-(c) and 6(d) and correspond to A, B, C and D in TABLE 2.

8

TABLE 2   INFLUENCE RENDERED TO POWDER SCATTERING BY FORMS OF AIR FLOW-IN OPENINGS

| FORM OF AIR FLOW-IN OPENINGS | POSITION OF AIR FLOW-IN OPENINGS | NO. OF SAMPLES n | RATE OF REMAINING IN VARIOUS PORTIONS (%) | | | RATE OF SCATTERING (%) |
|---|---|---|---|---|---|---|
| | | | INHALER | CAPSULE HEAD | CAPSULE BARREL | |
| A | inside | 3 | 8.45±0.33 | 0.26±0.03 | 1.99±0.09 | 89.3±0.76 |
| A | outside | 3 | 9.02±0.53 | 0.35±0.04 | 1.90±0.11 | 88.7±0.87 |
| B | inside | 3 | 15.4±1.22 | 0.30±0.02 | 6.40±0.82 | 77.9±1.30 |
| B | outside | 3 | 14.2±0.98 | 0.23±0.04 | 7.20±0.63 | 78.4±1.21 |
| C | inside | 3 | NO POWDER SCATTERED | | | 0 |
| C | outside | 3 | 17.3±1.83 | 0.42±0.06 | 7.71±1.23 | 74.6±0.71 |
| D | inside | 3 | 16.5±2.00 | 0.32±0.03 | 4.75±0.66 | 78.4±0.55 |
| D | outside | 3 | 16.9±2.19 | 0.45±0.04 | 4.01±0.18 | 78.6±0.81 |

As apparent from TABLE 2, the most satisfactory rate of scattering (rate of inhaling) was obtained by the form and arrangement of the air flow-in openings 5 shown in Fig. 6(a). In contrast thereto, the one shown in Fig. 6(c), wherein the air flow-in openings 5 are concentratedly arranged in the center of the end face of the capsule holding portion 2, shows the lowest rate of scattering. In the general tendency, the closer to the center the air flow-in openings 5 are arranged, the fewer the turbulent flows are formed, thus lowering the rate of scattering.

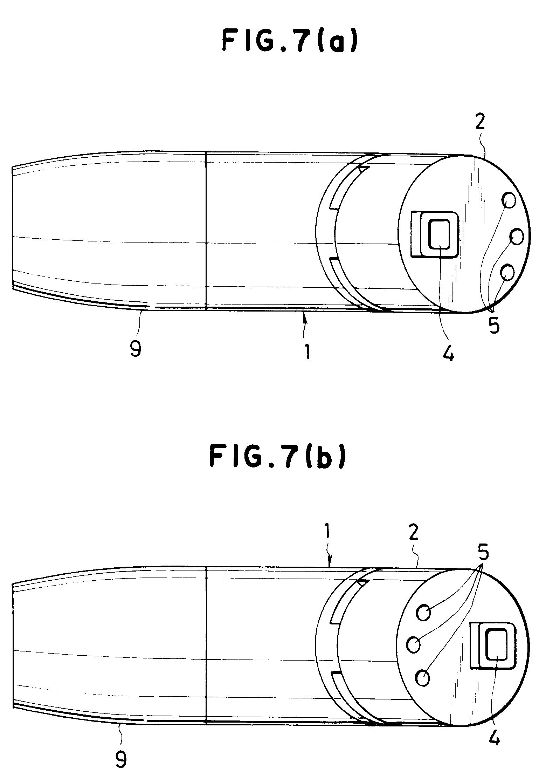

Further, the inventors of the present inventions conducted experiments on the influences rendered to the powder scattering by the positions of the air flow-in openings 5 relative to the hollow body 1. The results of experiments are also shown in TABLE 2. More specifically, an "outside" position and an "inside" position of the air flow-in openings in TABLE 2 indicate cases shown in Figs. 7(a) and 7(b), respectively. The "outside" position shows the case where inhaling is performed with the air flow-in openings 5 being positioned on the outer side of the bending direction of the bent section 1b of the hollow body 1 of the inhaler to the bent section 1b, and the "inside" position shows the case of the position contrary to the above.

According to TABLE 2, when the forms of the air flow-in openings are the ones shown in Figs. 6(a), 6(b) and 6(d), there is no much difference in rate of scattering between the "inside" position and the "outside" position. However, in the case of Fig. 6(c), when the air flow-in openings 5 are located at the "inside" position, the powder was not scattered at all.

When the inventors of the present invention ascertained by experiments the rates of powder scattering in the cases where the air flow-in openings 5 are located at the "inside" position, the "outside" position or the side positions for the L type, A type and S type inhalers in TABLE 1, respectively, the following results were obtained.

First, with the L type inhaler (shown in Fig. 5(a)), the operating manoeuvres of the capsule barrel were better when the air flow-in openings 5 were located at the "outside" position but not at the "inside" position. Furthermore, when the air flow-in openings were located at peripheral positions slightly inwardly of the "outside" position, i.e. the side position, satisfactory results were obtained.

Next, with the A type inhaler (shown in Fig. 5(b)), regardless of the positions of the air flow-in openings 5, the operating manoeuvres of the capsule barrel were good, a satisfactory scattering performance was obtained, and particularly, the "outside" position was excellent.

Furthermore, with the S type inhaler (shown in Fig. 5(c)), when the air flow-in openings 5 are located at the "outside" position, the operating manoeuvres of the capsule barrel were highly satisfactory, however, when the air flow-in openings 5 are located at the "inside" position, the operating manoeuvres of the capsule barrel were not briskly performed.

Consequently, putting all the above matters of studies together, it becomes apparent that the optimum requirements for the inhaler according to the present invention include the angle $\alpha$ formed by the axis of the hollow barrel section 1a of the hollow body 1 and the axis of the bent section 1b is 60 degrees, the length $\ell$ of the hollow barrel section 1a on the inner side in the bending direction is 2.5 - 3.0 cm, two air flow-in openings 5 of arcuate slit shapes are used with the opening area being about 0.31 cm$^2$, and the positions of the air flow-in openings 5 are on the outer side of the bent section 1b during inhaling.

[Embodiment 2]

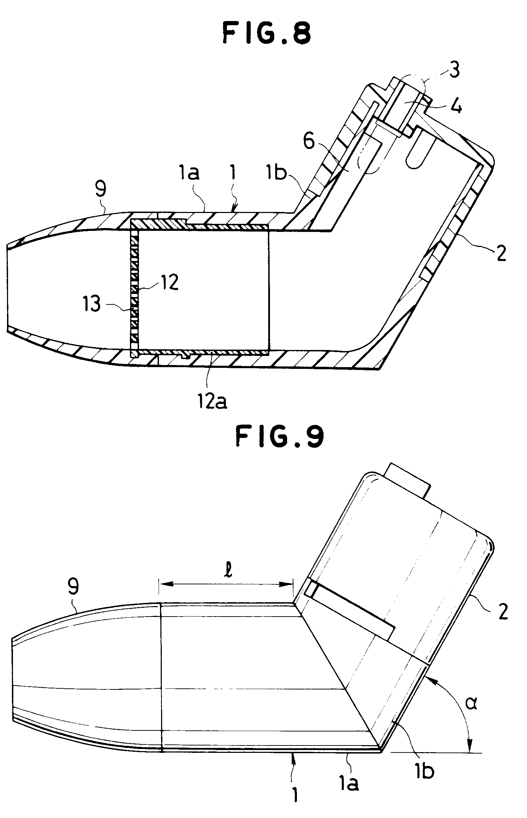

Figs. 8 and 9 are a sectional and a front views showing another embodiment of the inhaler according to the present invention.

In this embodiment 2, the porous plate 12 is integrally formed on a cylindrical portion 12a to provide a cup-shaped member, and the hollow barrel section 1a of the hollow body 1 and the inhaling section 9 are indirectly connected to each other through this cup-shaped member.

In the case of this embodiment, the inhaler as a whole is of a bent form, so that the powdered preparations can be prevented from leaking out of the inhaler when the capsule is separated, the operating manoeuvres of the capsule barrel can be improved and the rate of scattering of the powder, i.e. the rate of inhaling can be improved during inhaling.

Additionally, the present invention need not necessarily be limited to the above embodiments, and various other modifications can be adopted.

For example, in the above embodiments, the bent section 1b of the hollow body 1 is straight-lined, however, this may be curved.

Furthermore, the whole or a part of the inhaler may be made of a transparent or a semi-transparent synthetic resin material.

Further, the forms or arrangements of the air flow-in openings 5 may be changed to ones other than the above.

According to the present invention, the following outstanding advantages can be attained.

(1) The inhaler according to the present invention comprises the features as defined in claim 1:

The mentioned features make it possible that turbulent flows can be readily formed in the inhaler during inhaling, the operating manoeuvres of the capsule's portion in the inhaler can be improved, and

EP 0 271 029 B1

the material contained in the inhaler can be reliably inhaled into the oral cavity of the user from the inhaler without the material remaining in the capsule's portion and the inhaler.

(2) With the arrangement described in Item (1), the material contained in the capsule, which has fallen into the inhaler from the capsule, can be prevented from leaking to the outside of the inhaler when the capsule is divided into two.

(3) With the arrangement described in Item (1), the material contained in the capsule, which has fallen into the inhaler from the capsule, can be prevented from recirculating into the capsule head being open in the inhaler and remaining therein as it is during inhaling.

(4) With the arrangement described in Item (1), such a disadvantage can be obviated that the inhaler turns to fall down even when the inhaler is placed on a flat plane such as a table.

## Claims

1. An inhaler for use with a capsule (3) of non-pressurized contents comprising:

   a hollow body (1) including a hollow barrel section (1a) having a length ($\ell$) and a first central longitudinal axis, said hollow barrel section (1a) having opposite end portions, one of said end portions being a free end portion,

   a capsule holding portion (2) rotatably joined to said hollow body (1) and having an end face formed with a capsule insertion opening (4) and air intake openings (5), said capsule insertion opening (4) being intended to hold an appropriately sized capsule (3) of non-pressurized contents having a capsule head and a capsule barrel,

   abutting means (6) projecting from the inner surface of said hollow body (1), for abutting against the capsule barrel when the capsule holding portion is rotated a predetermined amount relative to the hollow body, so as to separate said capsule barrel from said capsule head,

   a hollow inhaling section (9) provided at the free end of the hollow barrel section (1a), and

   capsule discharge preventive means (12) formed with pores (13) to pass the non-pressurized capsule (3) contents therethrough, and for preventing said capsule barrel, which has fallen into said hollow body from being discharged to the outside of the inhaling section;

   **characterized** in that

   said hollow body (1) further includes a hollow bent section (1b) having an inner surface, and being connected to one end of said hollow barrel section (1a), said hollow bent section (1b) having a second central longitudinal axis inclined at an angle ($\alpha$) in the range of 30°-80° to the first central longitudinal axis of the hollow barrel section (1a) such that said hollow barrel section (1a) and said hollow bent section (1b) define a continuous internal flow path having a first flow path section along said first central longitudinal axis and a second flow path section along said second central longitudinal axis;

   said capsule holding portion (2) is rotatably joined to said bent section (1b) of said hollow body (1);

   said capsule head is held in the capsule insertion opening (4);

   said abutting means is projected from the inner surface of said hollow bent section (1b) of said hollow body (1) to permit free fall of the capsule barrel and the non-pressurized capsule (3) contents through the second flow path section into the first flow path section, when the hollow barrel section is held horizontally and the hollow bent section is orientated upwardly;

   said hollow inhaling section (9) has a suction communication with the first and second flow path sections of said internal flow path; and

   said capsule head held by said capsule insertion opening (4) is openend in said second flow path section and inclined to the first central longitudinal axis of said first flow path section.

2. An inhaler as set forth in claim 1 wherein the angle ($\alpha$) of the second central longitudinal axis of said bent section to the first central longitudinal axis of said hollow barrel section is 45°-60°.

3. An inhaler as set forth in claim 2 wherein the angle ($\alpha$) of the second central longitudinal axis of said bent section to the first central longitudinal axis of said hollow barrel section is 60°.

4. An inhaler as set forth in claim 1, wherein said air intake openings (5) are arcuate opening-shaped.

5. An inhaler as set forth in claim 4, wherein said air intake openings (5) are formed of two arcuate opening-shaped holes and the total opening area of said holes is about 0.3 cm$^2$.

6. An inhaler as set forth in claim 1, wherein said air intake openings (5) are generally capsule-shaped.

11

**7.** An inhaler as set forth in claim 6, wherein said generally slot-shaped air intake openings (5) are arranged arcuately with one another at positions close to the outer periphery of the end face of said capsule holding portion (2).

**8.** An inhaler as set forth in claim 1, wherein said air intake openings (5) are circular in shape.

**9.** An inhaler as set forth in claim 8, wherein said air intake openings (5) are formed of three circular holes and each of said holes is 1.5-4.0 mm in diameter.

**10.** An inhaler as set forth in claim 9, wherein said three circular air intake openings are arranged arcuately with one another at positions close to the outer periphery of an end face of said capsule holding portion (2) and each of said holes is about 3.5 mm in diameter.

**11.** An inhaler as set forth in claim 1, wherein said abutting means (6) is integrally formed on the inner surface of said bent section (1b).

**12.** An inhaler as set forth in claim 1, wherein a length frome one end of said hollow barrel section (1a) to a boundary portion between said hollow barrel section (1a) and said hollow bent section (1b) on the inner side in the bending direction of said hollow bent section (1b) is 1.5-3.5 cm.

**13.** An inhaler as set forth in claim 12, wherein the length from one end of said hollow barrel section (1a) to the boundary portion between said hollow barrel section (1a) and said bent section (1b) on the inner side in the bending direction of said bent section (1b) is about 2.5-3.0 cm.

**14.** An inhaler as set forth in claim 1, wherein said capsule discharging preventive means (12) is formed of a molded synthetic resin having a multitude of pores.

**15.** An inhaler as set forth in claim 1, wherein said inhaler is made of an opaque synthetic resin material.

**16.** An inhaler as set forth in claim 1 wherein said hollow bent section (1b) and said hollow barrel section (1a) define a continuous hollow chamber and permit development of a turbulent suction within said hollow chamber when mouth suction is applied to said inhaling section.

**17.** An inhaler as set forth in claim 1 wherein said inhaler is made of a transparent synthetic resin material.

**Patentansprüche**

**1.** Inhalator zur Verwendung mit einer Kapsel (3) mit drucklosem Inhalt, umfassend:
   einen Hohlkörper (1), der einen hohlen zylinderartigen oder hülsenartigen Abschnitt (1a) umfaßt, welcher eine Länge (ℓ) und eine erste mittlere Längsachse hat, wobei der hohle hülsenartige Abschnitt (1a) gegenüberliegende Endteile hat, von denen einer ein freier Endteil ist,
   einen Kapselhalteteil (2), der mit dem Hohlkörper (1) drehbar verbunden ist und eine Endfläche hat, die mit einer Kapseleinsetzöffnung (4) und Lufteinlaßöffnungen (5) gebildet ist, von denen die Kapseleinsetzöffnung (4) eine zweckentsprechend dimensionierte Kapsel (3) halten soll, wobei die Kapsel drucklosen Inhalt und einen Kapselkopf und einen Kapselkörper hat,
   Anschlagmittel (6), die von der Innenfläche des Hohlkörpers (1) vorragen, um sich gegen den Kapselkörper zu legen, wenn der Kapselhalteteil in einem vorbestimmten Ausmaß relativ zu dem Hohlkörper gedreht wird derart, daß der Kapselkörper von dem Kapselkopf getrennt wird,
   einen hohlen Inhalierabschnitt (9), der an dem freien Ende des hohlen hülsenartigen Abschnitts (1a) vorgesehen ist, und
   eine ein Austreten der Kapsel verhindernde Einrichtung (12), die mit Poren (13) versehen ist, um den drucklosen Inhalt der Kapsel (3) durch sie hindurchtreten zu lassen und um zu verhindern, daß der Kapselkörper, der in den Hohlkörper gefallen ist, zur Außenseite des Inhalierabschnitts abgegeben wird;
   dadurch **gekennzeichnet,** daß
   der Hohlkörper (1) weiterhin einen abgebogenen hohlen Abschnitt (1b) umfaßt, der eine Innenfläche hat und mit einem Ende des hohlen hülsenartigen Abschnitts (1a) verbunden ist, wobei der abgebogene hohle Abschnitt (1b) eine zweite mittlere Längsachse hat, die in einem Winkel ($\alpha$) im Bereich von 30° bis 80° zu der ersten mittleren Längsachse des hohlen hülsenartigen Abschnitts (1a) schräg

12

verläuft derart, daß der hohle hülsenartige Abschnitt (1a) und der abgebogene hohle Abschnitt (1b) einen kontinuierlichen inneren Strömungsweg bestimmen, der einen ersten Strömungswegabschnitt entlang der ersten mittleren Längsachse und einen zweiten Strömungswegabschnitt entlang der zweiten mittleren Längsachse hat;

der Kapselhalteteil (2) mit dem abgebogenen Abschnitt (1b) des Hohlkörpers (1) drehbar verbunden ist;

der Kapselkopf in der Kapseleinsetzöffnung (4) gehalten ist;

die Anschlageinrichtung von der inneren Fläche des abgebogenen hohlen Abschnitts (1b) des Hohlkörpers (1) vorragt, um freies Fallen des Kapselkörpers und des drucklosen Inhalts der Kapsel (3) durch den zweiten Strömungswegabschnitt in den ersten Strömungswegabschnitt zu ermöglichen, wenn der hohle hülsenartige Abschnitt horizontal und der abgebogene hohle Abschnitt aufwärts ausgerichtet sind;

der hohle Inhalierabschnitt (9) eine Saugverbindung mit dem ersten und dem zweiten Strömungswegabschnitt des inneren Strömungsweges hat; und

der Kapselkopf, der von der Kapseleinsetzöffnung (4) gehalten ist, in dem zweiten Strömungswegabschnitt geöffnet und zu der ersten mittleren Längsachse des ersten Strömungswegab schnitts schräggestellt wird.

2. Inhalator nach Anspruch 1, wobei der Winkel ($\alpha$) der Zweiten mittleren Längsachse des abgebogenen Abschnitts zu der ersten mittleren Längsachse des hohlen hülsenartigen Abschnitts 45° bis 60° beträgt.

3. Inhalator nach Anspruch 2, wobei der Winkel ($\alpha$) der zweiten mittleren Längsachse des abgebogenen Abschnitts zu der ersten mittleren Längsachse des hohlen hülsenartigen Abschnitts 60° beträgt.

4. Inhalator nach Anspruch 1, wobei die Lufteinlaßöffnungen (5) bogenförmige Öffnungsgestalt haben.

5. Inhalator nach Anspruch 4, wobei die Lufteinlaßöffnungen (5) aus zwei bogenförmig gestalteten Öffnungslöchern gebildet sind und der Gesamtöffnungsbereich der Löcher etwa 0,3 cm$^2$ beträgt.

6. Inhalator nach Anspruch 1, wobei die Lufteinlaßöffnungen (5) allgemein Kapselgestalt haben.

7. Inhalaltor nach Anspruch 6, wobei die allgemein schlitzförmigen Lufteinlaßöffnungen (5) bogenförmig zueinander an Positionen angeordnet sind, die nahe dem Außenumfang der Endfläche des Kapselhalteteils (2) liegen.

8. Inhalator nach Anspruch 1, wobei die Lufteinlaßöffnungen (5) Kreisgestalt haben.

9. Inhalator nach Anspruch 8, wobei die Lufteinlaßöffnungen (5) aus drei kreisförmigen Löchern gebildet sind und jedes Loch einen Durchmesser von 1,5 bis 4,0 mm hat.

10. Inhalator nach Anspruch 9, wobei die drei kreisförmigen Lufteinlaßöffnungen bogenförmig zueinander angeordnet sind an Positionen nahe dem Außenumfang einer Endfläche des Kapselhalteteils (2), wobei jedes der Löcher einen Durchmesser von etwa 3,5 mm hat.

11. Inhalator nach Anspruch 1, wobei die Anschlageinrichtung (6) an der Innenfläche des abgebogenen Abschnitts (1b) einheitlich gebildet ist.

12. Inhalator nach Anspruch 1, wobei die Länge von einem Ende des hohlen hülsenartigen Abschnitts (1a) zu einem Grenzabschnitt zwischen dem hohlen hülsenartigen Abschnitt (1a) und dem abgebogenen hohlen Abschnitt (1b) an der Innenseite in der Abbiegerichtung des abgebogenen hohlen Abschnitts (1b) 1,5 bis 3,5 cm beträgt.

13. Inhalator nach Anspruch 12, wobei die Länge von einem Ende des hohlen hülsenartigen Abschnitts (1a) zu dem Grenzabschnitt zwischen dem hohlen hülsenartigen Abschnitt (1a) und dem abgebogenen Abschnitt (1b) an der Innenseite in der Abbiegerichtung des abgebogenen Abschnitts (1b) etwa 2,5 bis 3,0 cm beträgt.

**14.** Inhalator nach Anspruch 1, wobei die ein Abgeben oder Austreten der Kapsel verhindernde Einrichtung (12) aus einem geformten synthetischen Harz gebildet ist, welches eine Mehrzahl von Poren hat.

**15.** Inhalaltor nach Anspruch 1, wobei der Inhalator aus einem undurchsichtigen oder lichtundurchlässigen synthetischen Harzmaterial gebildet ist.

**16.** Inhalator nach Anspruch 1, wobei der abgebogene hohle Abschnitt (1b) und der hohle hülsenartige Abschnitt (1a) eine kontinuierliche hohle Kammer bestimmen und Entwicklung eines turbulenten Saugstromes in der hohlen Kammer ermöglichen, wenn Mundansaugung an dem Inhalierabschnitt erfolgt.

**17.** Inhalator nach Anspruch 1, wobei der Inhalator aus einem transparenten synthetischen Harzmaterial gebildet ist.

**Revendications**

**1.** Un inhalateur à utiliser avec une capsule (3) dont le contenu n'est pas pressurisé, comprenant :

un corps creux (1) constitué d'une section cylindrique creuse (1a) ayant une longueur ($\ell$) et un premier axe central longitudinal cette section cylindrique creuse (1a) ayant des portions d'extrémité opposées, dont l'une est une portion d'extrémité libre,

une portion de retenue de capsule (2) assemblée au corps creux (1) et pouvant tourner par rapport à celui-ci, et ayant une face d'extrémité comportant une ouverture d'insertion de capsule (4) et des orifices d'arrivée d'air (5), l'ouverture d'insertion de capsule (4) ayant pour objet de retenir une capsule (3) de dimension appropriée dont le contenu n'est pas pressurisé, ayant une tête de capsule et un corps de capsule,

un moyen de butée (6) en saillie par rapport à la surface intérieure du corps creux (1), venant en butée contre le corps de capsule lorsque l'on fait tourner la portion de retenue de capsule d'une valeur prédéterminée par rapport au corps creux, de façon à séparer le corps de capsule de la tête de capsule,

une section d'inhalation creuse (9) placée à l'extrémité libre de la section cylindrique creuse (1a), et

un moyen s'opposant à la libération de la capsule (12) muni de pores (13) pour laisser passer le contenu non pressurisé de la capsule (3), et pour empêcher le corps de capsule tombé dans le corps creux d'être évacué à l'extérieur de la section d'inhalation ;

caractérisé en ce que

le corps creux (1) comprend en outre une section coudée creuse (1b) ayant une surface intérieure, et relié à une extrémité de la section cylindrique creuse (1a), cette section coudée creuse (1b) ayant un second axe central longitudinal incliné à un angle ($\alpha$) compris entre 30 et 80° par rapport au premier axe central longitudinal de la section cylindrique creuse (1a), de sorte que la section cylindrique creuse (1a) et la section coudée creuse (1b) définissent une trajectoire d'écoulement interne continu ayant une première section de trajectoire d'écoulement le long du premier axe central longitudinal et une seconde section de trajectoire d'écoulement le long du second axe central longitudinal ;

la portion de retenue de capsule (2) est assemblée à la section coudée (1b) du corps creux (1) et peut tourner par rapport à celle-ci ;

la tête de capsule est retenue par l'ouverture d'insertion de capsule (4) ;

le moyen de butée est en saillie par rapport à la surface intérieure de la section coudée creuse (1b) du corps creux (1) pour permettre au corps de capsule et au contenu non pressurisé de la capsule (3) de tomber librement dans la seconde section de trajectoire d'écoulement après être passés dans la première section de trajectoire d'écoulement, lorsque la section cylindrique creuse est tenue à l'horizontale et que la section coudée creuse est orientée vers le haut ;

la section d'inhalation creuse (9) a une communication d'aspiration avec les première et seconde sections de trajectoire d'écoulement de la trajectoire d'écoulement interne ; et

la tête de capsule retenue par l'ouverture d'insertion de capsule (4) est ouverte dans la seconde section de trajectoire d'écoulement et inclinée par rapport au premier axe central longitudinal de la première section de trajectoire d'écoulement.

**2.** Un inhalateur selon la revendication 1, où l'angle ($\alpha$) du second axe central longitudinal de la section coudée par rapport au premier axe central longitudinal de la section cylindrique creuse est de 45-60°.

**3.** Un inhalateur selon la revendication 2, où l'angle ($\alpha$) du second axe central longitudinal de la section cylindrique creuse est de 60°.

**4.** Un inhalateur selon la revendication 1, où les ouvertures d'arrivée d'air (5) ont une ouverture en arc.

**5.** Un inhalateur selon la revendication 4, où les ouvertures d'arrivée d'air (5) sont formées de deux trous ouverts en arc et où la surface d'ouverture totale de ces trous est d'environ 0,3 cm$^2$.

**6.** Un inhalateur selon la revendication 1, où les ouvertures d'arrivée d'air (5) ont une forme générale de capsule.

**7.** Un inhalateur selon la revendication 6, où les ouvertures d'arrivée d'air (5) de forme générale fendue sont disposées en arc les unes par rapport aux autres à des positions proches de la périphérie extérieure de la face d'extrémité de la portion de retenue de capsule (2).

**8.** Un inhalateur selon la revendication 1, où les ouvertures d'arrivée d'air (5) ont une forme circulaire.

**9.** Un inhalateur selon la revendication 8, où les ouvertures d'arrivée d'air (5) sont constituées de trois trous circulaires dont chacun a un diamètre d'1,5-4,0 mm.

**10.** Un inhalateur selon la revendication 9, où les trois ouvertures d'arrivée d'air circulaires sont disposées en arc les unes par rapport aux autres à des positions proches de la périphérie extérieure d'une face d'extrémité de la portion de retenue de capsule (2) et où chacun de ces trous a un diamètre d'environ 3,5 mm.

**11.** Un inhalateur selon la revendication 1, où le moyen de butée (6) fait partie intégrante de la surface intérieure de la section coudée (1b).

**12.** Un inhalateur selon la revendication 1, où une longueur d'une extrémité de la section cylindrique creuse (1a) à une portion limitrophe entre la section cylindrique creuse (1a) et la section coudée creuse (1b) sur la face intérieure dans le sens de courbure de la section coudée creuse (1b) est d'1,5-3,5 cm.

**13.** Un inhalateur selon la revendication 12, où la longueur d'une extrémité de la section cylindrique creuse (1a) à la portion limitrophe entre la section cylindrique creuse (1a) et la section coudée (1b) sur la face intérieure dans le sens de courbure de la section coudée (1b) est d'environ 2,5-3,0 cm.

**14.** Un inhalateur selon la revendication 1, où le moyen s'opposant à la libération de la capsule (12) est constitué d'une résine synthétique moulée ayant une multitude de pores.

**15.** Un inhalateur selon la revendication 1, où l'inhalateur est exécuté dans une résine synthétique opaque.

**16.** Un inhalateur selon la revendication 1, où la section coudée creuse (1b) et la section cylindrique creuse (1a) définissent une chambre creuse continue et permettent la création d'une aspiration turbulente à l'intérieur de cette chambre creuse lorsqu'une aspiration est appliquée par la bouche à la section d'inhalation.

**17.** Un inhalateur selon la revendication 1, où l'inhalateur est exécuté dans une résine synthétique transparente.

15

# FIG.1

FIG.2

# FIG.3

# FIG.4

# FIG.5(a)

# FIG.5(b)

# FIG.5(c)

FIG.6(a)

FIG.6(b)

FIG.6(c)

FIG.6(d)

## FIG.7(a)

## FIG.7(b)

# FIG.8

# FIG.9